Europäisches Patentamt

(19) European Patent Office  (11) Publication number: **0 165 529**

Office européen des brevets  **B1**

(12) **EUROPEAN PATENT SPECIFICATION**

(45) Date of publication of the patent specification:
25.11.87  (51) Int. Cl.⁴: **C 07 C 67/08,** C 07 C 69/54

(21) Application number: 85107003.7

(22) Date of filing: 05.06.85

(54) **Process for producing phenyl methacrylate or acrylate.**

(30) Priority: 06.06.84 JP 116163/84

(43) Date of publication of application:
27.12.85 Bulletin 85/52

(45) Publication of the grant of the patent:
25.11.87 Bulletin 87/48

(84) Designated Contracting States:
BE DE FR GB NL

(56) References cited:
US-A-3 106 570
US-A-4 258 204

(73) Proprietor: **MITSUBISHI RAYON CO. LTD., 3-19, Kyobashi 2-chome Chuo- Ku, Tokyo 104 (JP)**

(72) Inventor: **Hatakeyama, Hiroki, Mitsubishi Rayon Co., Ltd. No. 20- 1, Miyuki- cho, Otake- shi Hiroshima (JP)**
Inventor: **Takeda, Hitoshi, Mitsubishi Rayon Co., Ltd. No. 20- 1, Miyuki- cho, Otake- shi Hiroshima (JP)**

(74) Representative: **Patentanwälte TER MEER - MÜLLER - STEINMEISTER, Mauerkircherstrasse 45, D-8000 München 80 (DE)**

**Description**

The present invention relates to a process for producing a phenyl ester of methacrylic acid or acrylic acid (hereinafter referred to as (meth)acrylic acid).

Phenyl esters of (meth)acrylic acid are useful monomers which can be polymerized to provide polymers having a high refractive index, a low hygroscopicity and a high thermal resistance. It is generally considered that they cannot be synthesized directly from a carboxylic acid and a phenol, since the reaction equilibrium inclines greatly toward the hydrolysis.

Therefore, phenyl (meth)acrylate has previously been synthesized from (meth)acryloyl chloride by a process comprising complicated steps consequently leading to an expensive product.

A process wherein the carboxylic acid per se is used as a starting material is disclosed in JACS 75, 5416 (1953). According to this process, phenyl methacrylate is synthesized from methacrylic acid and phenol by using polyphosphoric acid as a dehydrating agent. In this process, however, polyphosphoric acid is used in the high amount of 2/3 part by weight polyphosphoric acid per part by weight of phenol and the yield is as low amounting only up to 55 %. This process is thus industrially not advantageous.

Under the above-described circumstances it is therefore an object of the present invention to provide a process for producing useful phenyl (meth)acrylate efficiently and at low costs.

This object is achieved by the process according to claim 1. The subclaims relate to preferred embodiments of this process.

The process of the present invention for producing a phenyl (meth)acrylate is characterized in that (meth)acrylic acid is subjected to a dehydration reaction with a compound having a phenolic hydroxyl group in the presence of an acid catalyst at a temperature of at least 110°C.

The compounds having a phenolic hydroxyl group usable in the process of the present invention include, for example, phenol, p-chlorophenol, 2,3,5-trichlorophenol, pentachlorophenol, pentabromophenol, p-bromophenol, p-methoxyphenol, o-methoxyphenol, p-benzylphenol, p-phenylphenol, o-phenylphenol, p-phenoxyphenol, p-ethoxyphenol, o-butoxyphenol, p-nitrophenol, p-cyanophenol, p-hydroxybenzenesulfonic acid, p-hydroxystyrene, o-cresol, 2-hydroxy-p-xylene, p-methoxymethylphenol, o-methoxymethylphenol, p-t-butylphenol, p-toluylphenol, p-tetrahydrofurfurylphenol, 6-hydroxyquinoline and 2-hydroxytetralin. The compounds having a phenolic hydroxyl group are not particularly limited provided that they do not comprise a group which hinders the reaction. The amount of the compound having a phenolic hydroxyl group is also not particularly limited. However, when this compound is more expensive than the (meth)acrylic acid, it is used preferably in an amount of up to 1 equivalent, and particularly in an amount of 0.2 to 1.0 equivalents per equivalent of (meth)acrylic acid. When the former compound is less expensive than the latter compound, the former is used preferably in an amount of at least 1 equivalent and particularly in an amount of 1.0 to 4.0 equivalents per equivalent of the latter. The use of the reactants in such amounts is effective for the production of the intended phenol esters at low costs.

According to the process of the present invention, the dehydration reaction is carried out at a temperature of at least 110°C, wherein preferably a solvent is used. The solvent used is not particularly limited provided it has a boiling point of at least 110°C and is inert and immiscible with water. Even a solvent having a boiling point of less than 110°C can be used in such a small amount that the reaction temperature can be kept at 110°C or higher. Solvents having a boiling point lower than that of acrylic or methacrylic acid are preferred. Examples usable solvents include toluene, xylene, chlorobenzole, n-octane and n-nonane. The amount of the solvent is such that the dehydration reaction proceeds satisfactorily and is therefore not particularly limited.

The dehydration reaction according to the process of the present invention is carried out at a temperature of at least 110°C, preferably of more than 125°C, and particularly in the range of 125°C to the boiling point of (meth)acrylic acid. When a reaction temperature higher than the boiling point of (meth)acrylic acid is used, an unfavourable polymerization of (meth)acrylic acid is caused. On the other hand a practical reaction rate cannot be obtained at a temperature of below 110°C. Preferably the reaction is carried out under atmospheric pressure so as to avoid the use of a complicated apparatus even though the reaction may be also carried out under reduced pressure or elevated pressure. The reaction time, which varies with the reaction temperature, the catalyst and the starting compound having a phenolic hydroxyl group, comprises usually 5 to 30 h. When an alcohol different from the compound having a phenolic hydroxyl group is used for the esterification under the conditions (reaction temperature and reaction time) of the present invention, (meth)acrylic acid or the phenyl (meth)acrylate is polymerized inevitably. Thus, the reaction conditions employed in the present invention are specific ones to be employed for the reaction of only the compound having a phenolic hydroxyl group.

The acid catalysts used in the present invention are those used generally for esterification reactions. Particularly preferred are sulfuric acid, a combination of sulfuric acid and boric acid or p-toluenesulfonic acid.

The amount of the catalyst may be equal to the amount generally used in esterification reactions. This amount generally is 0.1 to 30 wt. % based on (meth)acrylic acid.

Though the use of a polymerization inhibitor which does not participate in reactions similar to that of the present invention is generally preferred to inhibit the polymerization, the dehydration according to the present invention can be carried out without using a polymerization inhibitor by introducing air into the reaction system.

The catalyst used, the unreacted (meth)acrylic acid and the unreacted compound having a phenolic hydroxyl group can be removed easily from the reaction product by washing it with an aqueous alkali solution after completion of the reaction. By a distillation or recrystallization conducted after the washing, the colorless, transparent phenyl (meth)acrylate is obtained.

The following examples and comparative examples further illustrate the present invention but shall by no means limit the present invention to these embodiments. In the examples and comparative examples, parts and percentages are given by weight.

## Example 1

103 parts of methacrylic acid, 75 parts of phenol, 2.4 parts of boric acid, 1.9 parts of sulfuric acid and 170 parts of xylene are mixed together and the reaction is carried out at 150°C for 10 h while air is introduced and the dehydration is effected by xylene azeotropy. The resulting reaction liquid is washed three times with 300 parts of 1.5 N aqueous sodium hydroxide solution and then distilled to obtain 94.7 parts (yield: 73 %) of colorless, transparent phenyl methacrylate. No polymerization ocourred in the course of the reaction.

## Example 2

The reaction is carried out at 150°C for 15 h in the same manner as in Example 1 except that 5.9 parts of sulfuric acid are used as the catalyst to obtain 88.2 parts (yield: 68 %) of phenyl methacrylate.

## Example 3

The reaction is carried out at 125°C for 22 h in the same manner as in Example 1 except that 173 parts of toluene are used as the solvent to obtain 81.7 parts (yield: 63 %) of phenyl methacrylate.

## Example 4

The reaction is carried out at 150°C for 15 h in the same manner as in Example 1 except that 11.4 parts of p-toluenesulfonic acid are used as the catalyst to obtain 77.9 parts (yield: 60 %) of phenyl methacrylate.

## Example 5

The reaction is carried out in the same manner as in Example 3 except that methacrylic acid is replaced by 86 parts of acrylic acid. 77 parts (yield: 65 %) of phenyl acrylate are obtained.

## Examples 6 to 12

The reaction is carried out in the same manner as in Example 1 using the phenol shown in the following table providing the yields shown therein as well:

|  | Phenol | Yield |
|---|---|---|
| Example 6 | p-methoxyphenol | 68 % |
| Example 7 | β-naphthol | 38 % |
| Example 8 | p-chlorophenol | 83 % |
| Example 9 | guaiacol | 54 % |
| Example 10 | o-phenylphenol | 48 % |
| Example 11 | p-bromophenol | 63 % |
| Example 12 | p-t-butylphenol | 66 % |

Comparative Example 1
The reaction is carried out in the same manner as in Example 1 except that phenol is replaced by 2-ethylhexanol. About two hours after initiation of the reaction, the polymerization occurred.

**0 165 529**

Comparative Example 2

The reaction is carried out at 95°C for 40 h in the same manner as in Example 1 except that cyclohexane is used as the solvent. Only 16.7 parts of phenyl methacrylate are obtained (yield: 13 %)

According to the process of the present invention, a phenyl (meth)acrylate can be synthesized efficiently by subjecting (meth)acrylic acid and a compound having a phenolic hydroxyl group to a dehydration reaction at a temperature of at least 110°C in the presence of a catalyst. The process of the invention is quite advantageous for the production of the phenyl (meth)acrylate having excellent properties such as a high refractive index, low hygroscopicity and high thermal resistance on an industrial scale. According to this process, phenyl (meth)acrylate can be obtained at costs far lower than that required in the conventional process wherein (meth)acryloyl chloride is used as the starting material.

**Claims**

1. A process for producing a phenyl methacrylate or acrylate, <u>characterized</u> in that methacrylic acid or acrylic acid is subjected to a dehydration reaction with a compound having a phenolic hydroxyl group in the presence of an acid catalyst at a temperature of at least 110°C.

2. The process of claim 1, <u>characterized</u> in that the dehydration reaction is carried out while air is introduced in the reaction system.

3. The process of claim 1, <u>characterized</u> in that the acid catalyst used is sulfuric acid.

4. The process of claim 1, <u>characterized</u> in that the acid catalyst used is a combination of sulfuric acid and boric acid.

5. The process of claim 1, <u>characterized</u> in that the acid catalyst used is p-toluenesulfonic acid.

6. The process of claim 1, <u>characterized</u> in that the temperature is above 125°C.

7. The process of claim 1, <u>characterized</u> in that the temperature is in the range of 125°C to the boiling point of (meth)acrylic acid.

**Patentansprüche**

1. Verfahren zur Herstellung eines Phenylmethacrylats oder -acrylats, <u>dadurch gekennzeichnet</u>, daß man Methacrylsäure oder Acrylsäure einer Dehydratisierung mit einer eine phenolische Hydroxylgruppe aufweisenden Verbindung in Gegenwart eines Säurekatalysators bei einer Temperatur von mindestens 110°C unterwirft.

2. Verfahren nach Anspruch 1, <u>dadurch gekennzeichnet</u>, daß man die Dehydratisierung durchführt, währenddem Luft in das Reaktionssystem eingeleitet wird.

3. Verfahren nach Anspruch 1, <u>dadurch gekennzeichnet</u>, daß man als Säurekatalysator Schwefelsäure einsetzt.

4. Verfahren nach Anspruch 1, <u>dadurch gekennzeichnet</u>, daß man als Säurekatalysator eine Kombination aus Schwefelsäure und Borsäure einsetzt.

5. Verfahren nach Anspruch 1, <u>dadurch gekennzeichnet</u>, daß man als Saurekatalysator p-Toluolsulfonsäure einsetzt.

6. Verfahren nach Anspruch 1, <u>dadurch gekennzeichnet</u>, daß die Temperatur oberhalb von 125°C liegt.

7. Verfahren nach Anspruch 1, <u>dadurch gekennzeichet</u>, daß die Temperatur im Bereich von 125°C bis zum Siedepunt der (Meth)acrylsäure liegt.

**Revendications**

1. Un procédé de préparation de méthacrylate ou acrylate de phényle, caractérisé en ce que l'on soumet l'acide méthacrylique ou l'acide acrylique à une réaction de déshydratation avec un composé portant un groupe hydroxy phénolique en présence d'un catalyseur acide à une température d'au moins 110°C.

2. Le procédé de la revendication 1, caractérisé en ce que la réaction de déshydratation est effectuée sous introduction d'air dans le système de réaction.

3. Le procédé de la revendication 1, caractérisé en ce que le catalyseur acide utilisé est l'acide sulfurique.

4. Le procédé de la revendication 1, caractérisé en ce que le catalyseur acide utilisé consiste en une combinaison d'acide sulfurique et d'acide borique.

5. Le procédé de la revendication 1, caractérisé en ce que le catalyseur acide utilisé est l'acide p-toluène sulfonique.

6. Le procédé de la revendication 1, caractérisé en ce que la température est supérieure à 125°C.

7. Le procédé de la revendication 1, caractérisé en ce que la température se situe dans l'intervalle allant de 125°C jusqu'au point d'ébullition de l'acide (méth)acrylique.

4